(19) Europäisches Patentamt
European Patent Office
Office européen des brevets

(11) Veröffentlichungsnummer: **0 096 813 B1**

(12) # EUROPÄISCHE PATENTSCHRIFT

(45) Veröffentlichungstag der Patentschrift: **27.12.85**

(21) Anmeldenummer: **83105501.7**

(22) Anmeldetag: **03.06.83**

(51) Int. Cl.⁴: **C 07 C 45/51**, C 07 C 49/255, C 07 C 43/225, C 07 C 41/16, C 07 C 79/36

(54) **Verfahren zur Herstellung von 5-Aryloxy-1-chlor-3,3-dimethyl-pentan-2-onen.**

(30) Priorität: **12.06.82 DE 3222221**

(43) Veröffentlichungstag der Anmeldung: **28.12.83 Patentblatt 83/52**

(45) Bekanntmachung des Hinweises auf die Patenterteilung: **27.12.85 Patentblatt 85/52**

(84) Benannte Vertragsstaaten: **BE CH DE FR GB IT LI**

(56) Entgegenhaltungen:
**EP - A - 0 055 427**
**DE - A - 3 021 581**

**TETRAHEDRON LETTERS, Band 22, Nr. 8, 1981, Seiten 759-762, Pergamon Press Ltd., GB, M. SUDA u.a.: "Dichloromethylenation of esters and lactones by triphenylphosphine-carbon tetrachloride reagent"**

(73) Patentinhaber: **BAYER AG, Konzernverwaltung RP Patentabteilung, D-5090 Leverkusen 1 Bayerwerk (DE)**

(72) Erfinder: **Jautelat, Manfred, Dr., Müllersbaum 28, D-5093 Burscheid (DE)**
Erfinder: **Arlt, Dieter, Prof. Dr., Rybnikerstrasse 2, D-5000 Köln 80 (DE)**
Erfinder: **Jäger, Gerhard, Dr., Gellertstrasse 18, D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Die vorliegende Erfindung betrifft ein neues Verfahren zur Herstellung von teilweise bekannten 5-Aryloxy-1-chlor-3,3-dimethyl-pentan-2-onen, die als Zwischenprodukte für die Synthese von fungizid wirksamen Azol-Derivaten verwendet werden können.

Es ist bereits bekannt geworden, daß man Chlormethylketone selektiv aus 1,1-Dichlor-1-alkenen durch Umsetzung mit Phenolaten und saure Hydrolyse erhält (Deutsche Offenlegungsschrift 3 049 461 [Le A 20 764]). Dieses Verfahren ist breit anwendbar und erfährt seine Einschränkungen im wesentlichen in der Zugänglichkeit der 1,1-Dichlor-1-alkene. 1,1-Dichlor-1-alkene werden durch Umsetzung von geeigneten Alkylchloriden und Vinylidenchlorid hergestellt (J. Am. Chem. Soc. 74, 2885 [1952]). Die zur Herstellung von 5-Aryloxy-1,1-dichlor-3,3-dimethyl-1-pentenen erforderlichen Ausgangsstoffe, 4-Aryloxy-2-chlor-2-methyl-butane, sind jedoch nur sehr schwierig zugänglich. So findet man bei der Umsetzung von 2,4-Dichlor-2-methylbutan mit Phenolaten Eliminierung zu Olefinen.

Es wurde nun gefunden, daß man 5-Aryloxy-1-chlor-3,3-dimethyl-pentan-2-one der Formel

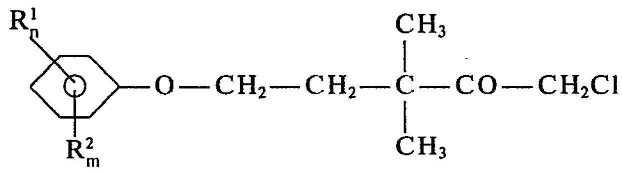

(I)

in welcher

$R^1$ und $R^2$ gleich oder verschieden sind und für Halogen, Alkyl mit 1 bis 4 Kohlenstoffatomen, Alkoxy und Alkylthio mit jeweils 1 bis 2 Kohlenstoffatomen, Halogenalkyl, Halogenalkoxy und Halogenalkylthio mit jeweils 1 bis 2 Kohlenstoffatomen und 1 bis 5 gleichen oder verschiedenen Halogenatomen stehen, für Cyclohexyl, für Dialkylamino mit 1 bis 4 Kohlenstoffatomen in jedem Alkylteil und weiterhin für Nitro, Cyano, sowie Alkoxycarbonyl mit 1 bis 4 Kohlenstoffatomen im Alkylteil und für gegebenenfalls durch Halogen substituiertes Phenyl stehen, und

n und m gleich oder verschieden sind und für die Werte 0, 1, 2, 3, 4 oder 5 stehen, mit der Einschränkung, daß die Summe von n und m nicht größer als 5 ist,

erhält, wenn man 1,1,5-Trichlor-3,3-dimethyl-1-penten der Formel

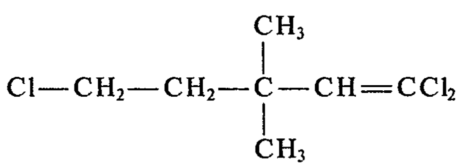

(II)

mit Phenolen der Formel

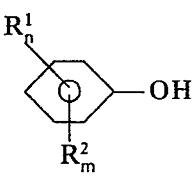

(III)

in welcher
$R^1$, $R^2$, n und m die obige Bedeutung haben,
in Gegenwart einer Base in einem Verdünnungsmittel zu Verbindungen der Formel

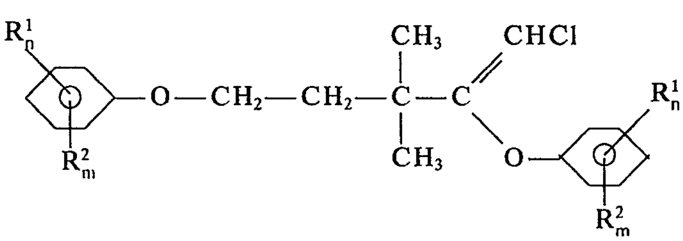

(IV)

in welcher
$R^1$, $R^2$, n und m die obige Bedeutung haben,
umsetzt und dieses Reaktionsprodukt einer sauren Hydrolyse unterwirft.

Da es bekannt ist, daß die Umsetzung von 1,1,5-Trichlor-3,3-dimethyl-1-penten (II) mit Alkoholaten

unter Eliminierung zu 3,3-Dimethylpent-4-en-säure-Derivaten führt (Deutsche Offenlegungsschrift 3 029 270 [Le A 20 457]), ist es als ausgesprochen überraschend zu bezeichnen, daß nach dem erfindungsgemäßen Verfahren mit Phenolen der Formel (III) in Gegenwart einer Base keine Eliminierung, sondern Substitution zu 5-Aryloxy-Derivaten der Formel (IV) erfolgt.

Das erfindungsgemäße Verfahren weist eine Reihe von Vorteilen auf. So ist der Ausgangsstoff der Formel (II), 1,1,5-Trichlor-3,3-dimethyl-1-penten, aus Isopren, Chlorwasserstoff und Vinylidenchlorid einfach zugänglich. Mit diesem zentralen Einsatzstoff und verschiedenen Phenolen lassen sich zahlreiche neue Diaryloxy-Verbindungen der Formel (IV) herstellen. Nach saurer Hydrolyse der Diaryloxy-Verbindungen der Formel (IV) stehen 5-Aryloxy-1-chlor-3,3-dimethyl-pentan-2-one (I) in guter Ausbeute zur Verfügung.

Verwendet man beispielsweise 1,1,5-Trichlor-3,3-dimethyl-1-penten und 2,4-Dichlorphenol als Ausgangsstoffe, so kann die Reaktion durch das folgende Formelschema wiedergegeben werden:

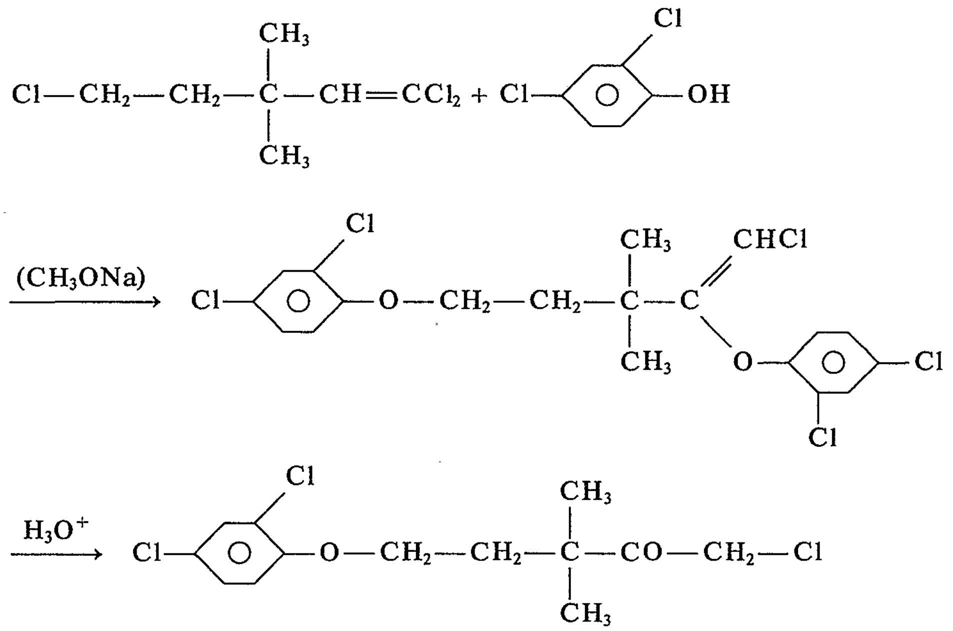


Das als Ausgangsstoff verwendete 1,1,5-Trichlor-3,3-dimethyl-1-penten ist durch die Formel (II) eindeutig bezeichnet. Die Verbindung und deren Herstellung ist bekannt (vgl. hierzu die Deutsche Offenlegungsschrift 3 029 270 [Le A 20 475] und obige Angaben).

Die weiterhin erfindungsgemäß zu verwendenden Phenole sind durch die Formel (III) allgemein definiert. In dieser Formel stehen $R^1$ und $R^2$ vorzugsweise für Halogen, für Alkyl, Alkoxy und Alkylthio mit jeweils 1 bis 2 Kohlenstoffatomen, für Halogenalkyl, Halogenalkoxy und Halogenalkylthio mit 1 bis 2 Kohlenstoff- und 1 bis 5 Fluor- oder Chloratomen, für Cyclohexyl, für Dialkylamino mit 1 bis 2 Kohlenstoffatomen in den Alkylteilen, für Nitro und Cyano, weiterhin für Alkoxycarbonyl mit 1 bis 2 Kohlenstoffatomen im Alkylteil und für gegebenenfalls durch Chlor oder Fluor ein- bis dreifach substituiertes Phenyl, und die Indizes n und m stehen vorzugsweise für die Zahlen 0, 1 und 2.

Die Phenole der Formel (III) sind bekannte und laboratoriumsübliche Verbindungen. Als Beispiele sind zu nennen:

Phenol, 2-Chlorphenol, 4-Chlorphenol, 3-Chlorphenol, 2,4-Dichlorphenol, 2,5-Dichlorphenol, 2,6-Dichlorphenol, 2,4,5-Trichlorphenol, 2,4,6-Trichlorphenol, Pentachlorphenol, 2-Nitrophenol, 4-Nitrophenol, 3-Nitrophenol, 4-Chlor-2-nitrophenol, 3-Chlor-4-nitro-phenol, 2,4-Dichlor-5-nitrophenol, 2-Methylphenol, 3-Methylphenol, 4-Methylphenol, 4-Chlor-2-methylphenol, 5-Chlor-2-methylphenol, 2-Methyl-5-nitrophenol, 4-Chlor-3-methylphenol, 3-Methyl-4-nitrophenol, 4-Methyl-2-nitrophenol, 3,5-Dimethylphenol, 4-tert.-Butylphenol, 4-Cyclohexylphenol, 4-Phenylphenol, 4-Fluorphenol, 2-Fluorphenol, 2,4-Difluorphenol, 2-Chlor-4-fluorphenol, 4-Trifluormethylthio-phenol, 4-Trifluormethoxy-phenol, 3-Trifluormethyl-phenol, 2-Chlor-4-trifluormethyl-phenol, 4-Cyanophenol.

Als Verdünnungsmittel kommen alle inerten organischen Lösungsmittel in Frage. Vorzugsweise werden polare Lösungsmittel, insbesondere Dimethylformamid, N-Methylpyrrolidon, Hexamethylphosphorsäuretriamid, Dimethylsulfoxid, 1,3-Dimethyl-2-imidazolidon, Tetramethylharnstoff, Sulfolan, Ether, wie Diethylether, Dioxan oder Alkohole, wie Glykol, Diethylenglykolmonoethylether oder Diethylenglykolmonomethylether unter anderem verwendet.

Die Umsetzung des 1,1,5-Trichlor-3,3-dimethyl-1-pentens (II) mit Phenolen wird in Gegenwart von Basen vorgenommen. Als solche kommen Alkali- und Erdalkalihydroxide wie Natriumhydroxid, Kaliumhydroxid oder Calciumhydroxid, Alkali- und Erdalkalicarbonate, wie Kaliumcarbonat oder Natriumcarbonat sowie Alkalialkoholate, wie Natriummethoxid, Natriumethoxid, Natriumbutoxid und Kalium-tert.-butoxid in Frage.

Die Reaktionstemperatur kann bei der Umsetzung von der Verbindung der Formel (II) mit den Phenolen der Formel (III) in einem größeren Bereich variiert werden. Im allgemeinen arbeitet man zwischen +100 und 250°C, vorzugsweise zwischen 120 und 220°C. Die Umsetzung kann sowohl bei Normaldruck im offenen System als auch unter Druck im Autoklaven im Druckbereich zwischen 1 und 50 bar, ausgeführt werden.

Bei der Durchführung des erfindungsgemäßen Verfahrens setzt man 2 bis 4 Äquivalente, vorzugsweise 2 bis 3, Phenol der Formel (III) mit 2 bis 4 Äquivalenten Base, vorzugsweise 2 bis 3, mit einem Äquivalent 1,1,5-Trichlor-3,3-dimethyl-1-penten (II) zu (IV) um.

Die nachfolgende Hydrolyse wird mit Mineralsäuren, vorzugsweise Schwefelsäure oder Salzsäure, und/oder mit organischen Säuren, wie Ameisensäure, Trifluoressigsäure, Oxalsäure, p-Toluolsulfonsäure oder Methansulfonsäure bei Temperaturen von +20 bis 150°C, vorzugsweise bei 40 bis 100°C, ausgeführt. Als Lösungsmittel kommen für die Hydrolyse Alkohole, wie Ethanol oder Methanol, Ketone, wie Aceton, oder Ether, wie Dioxan in Betracht. Die Säuren werden im allgemeinen im Überschuß eingesetzt. Sie können auch in wäßriger Verdünnung vorliegen. Gemäß einer besonderen Ausführungsform kann so verfahren werden, daß man zunächst das 1,1,5-Trichlor-3,3-dimethyl-1-penten der Formel (II) mit nur einem Äquivalent eines Phenols der Formel (III) in Gegenwart einer Base zu einer Verbindung der Formel

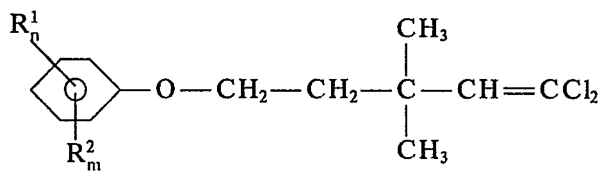

(V)

in welcher
$R^1$, $R^2$, n und m die obige Bedeutung haben,
umsetzt und in einer zusätzlichen Stufe mit einem Phenolat der Formel

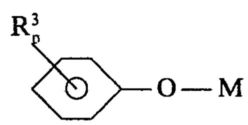

(VI)

in welcher

$R^3$ für Halogen und für Alkyl mit 1 bis 2 Kohlenstoffatomen steht,
p für die Zahlen 0, 1, 2 oder 3 steht, und
M für ein Äquivalent eines Alkali- oder Erdalkalimetallions steht,

zur Reaktion bringt, wobei man zu Verbindungen der Formel

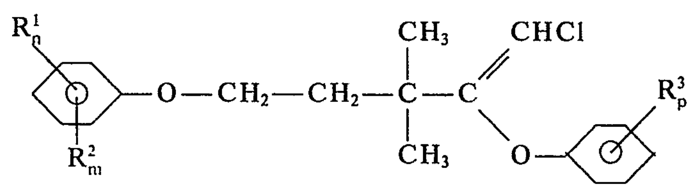

(VII)

in welcher
$R^1$, $R^2$, $R^3$, n, m und p die weiter oben angegebene Bedeutung besitzen,
gelangt, die dann verfahrensgemäß der sauren Hydrolyse unterworfen werden, wobei man die Verbindungen der Formel (I) erhält. Diese Verfahrensvariante ist insbesondere dann vorteilhaft, wenn die Phenole der Formel (II) teuer oder schwer zugänglich sind; als Phenolate der Formel (VI) wird man dann zweckmäßigerweise großtechnisch leicht zugängliche Verbindungen wählen.

Eine weitere besondere Ausführungsform besteht darin, daß man die üblicherweise getrennten Umsetzungen (a) von 1,1,5-Trichlor-3,3-dimethyl-1-penten der Formel (II) mit einem Phenol der Formel (III) und (b) die Hydrolyse von den Verbindungen der Formel (IV) in einem Eintopfverfahren zusammenfaßt.

Die Verbindungen der Formel (I) sind interessante Zwischenprodukte; sie sind zum Teil bekannt (Deutsche Offenlegungsschrift 3 049 461 [Le A 20 764]). Durch Umsetzung mit Azolen der Formel

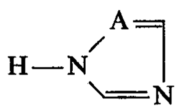

(VIII)

in welcher
A für Stickstoff oder die CH-Gruppe steht,
in Gegenwart eines Säurebindemittels und eines Verdünnungsmittels erhält man Verbindungen der Formel

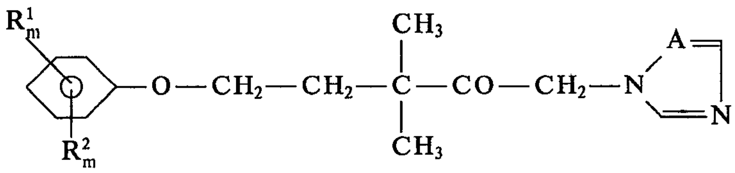

(IX)

in welcher
$R^1$, $R^2$, n, m und A die oben angegebene Bedeutung besitzen,
die sich mit Propargylhalogeniden in Gegenwart von aktiviertem Aluminium zu Hydroxyalkyl-azolyl-Derivaten der Formel

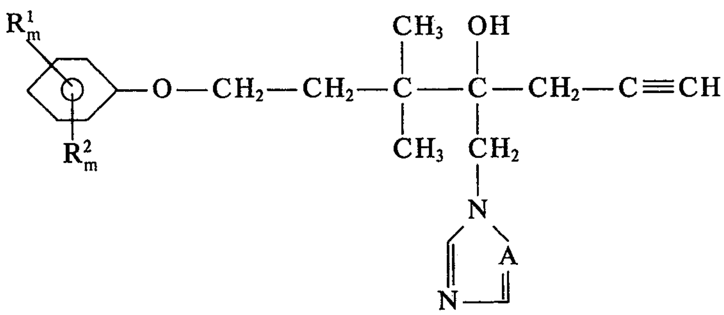

(X)

in welcher
$R^1$, $R^2$, n, m und A die oben angegebene Bedeutung besitzen,
umsetzen lassen. Die Verbindungen besitzen fungizide Wirksamkeit.

## Herstellungsbeispiele

### Beispiel 1

#### 1. Stufe

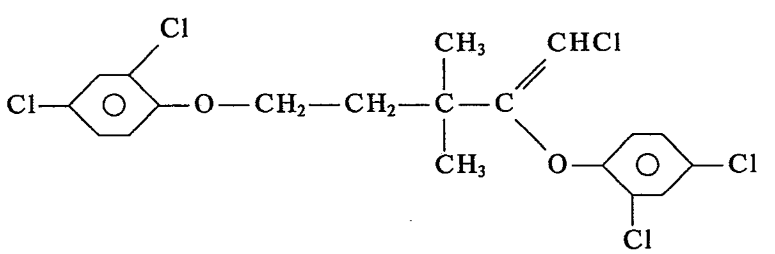

489 g (3 Mol) 2,4-Dichlorphenol werden in 1,2 l N-Methylpyrrolidon gelöst und mit 600 ml (3 Mol) einer 30%igen Natriummethylatlösung versetzt. Bei 20 mbar destilliert man Methanol und 200 ml N-Methylpyrrolidon ab. 201 g (1 Mol) 1,1,5-Trichlor-3,3-dimethyl-1-penten werden bei 200°C und Atmosphärendruck langsam eingetropft. Man rührt 6 Stunden bei 200°C nach. Die erkaltete Lösung wird mit Methylenchlorid verdünnt und mit verdünnter Natronlauge mehrfach ausgeschüttelt. Die getrocknete Lösung wird am Rotationsverdampfer bei 0,1 mbar und 150°C vom Lösungsmittel befreit. Es bleiben 410 g (0,9 Mol; 90% der Theorie) 1-Chlor-3,3-dimethyl-2,5-di-(2,4-dichlorphenoxy)-1-penten als Öl zurück.

NMR ($CDCl_3$): $\delta =$ 1,2 (s, 6 H), 2,1 (t, 2 H, J = 7 Hz), t, 2 H, J = 7 Hz), 5,95 (s, 1 H), 6,75–7,4 (m, 6 H).

2. Stufe

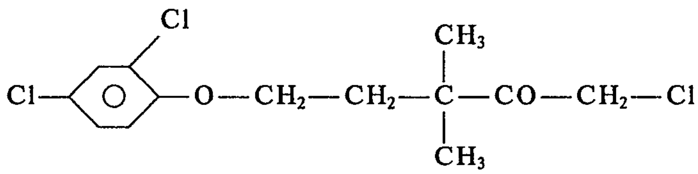

454 g (1 Mol) 1-Chlor-3,3-dimethyl-2,5-di-(2,4-dichlorphenoxy)-1-penten werden in 500 ml Ameisensäure und 50 ml konzentrierter Salzsäure während 9 Stunden auf 100°C erhitzt. Man verdünnt mit Methylenchlorid, schüttelt einmal mit Wasser und dreimal mit verdünnter Natronlauge aus. Nach dem Trocknen der Lösung wird das Lösungsmittel im Vakuum abgezogen. Es bleiben 238 g (0,77 Mol; 77% der Theorie) 5-(2,4-Dichlorphenoxy)-1-chlor-3,3-dimethylpentan-2-on zurück, das langsam kristallisiert. Nach Waschen mit Petrolether liegt der Schmelzpunkt bei 55—58°C.

Beispiel 2

1. Stufe

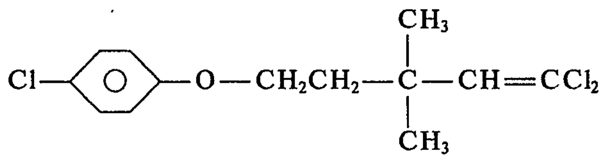

128 g (1 Mol) 4-Chlorphenol, 201 g (1 Mol) 1,1,5-Trichlor-3,3-dimethyl-1-penten (II) und 138 g (1 Mol) Kaliumcarbonat werden in 1 l Diethylenglykol-dimethylether 10 Stunden unter Rückfluß erhitzt. Dann wird mit Methylenchlorid verdünnt und mit Wasser mehrfach ausgeschüttelt. Die organische Phase wird am Rotationsverdampfer vom Lösungsmittel befreit und anschließend bei Kp 0,1 mbar/135—140°C destilliert. Man erhält 252 g (0,86 Mol, das sind 86% der Theorie) 5-(4-Chlorphenoxy)-1,1-dichlor-3,3-dimethyl-1-penten.

NMR ($CDCl_3$): $\delta =$ 1,2 (s, 6 H), 1,95 (t, 2 H, J = 7 Hz) 4,0 (t, 2 H), J = 7 Hz) 6,0 (s, 1 H), 6,75—7,25 ppm (m, 4 H).

2. Stufe

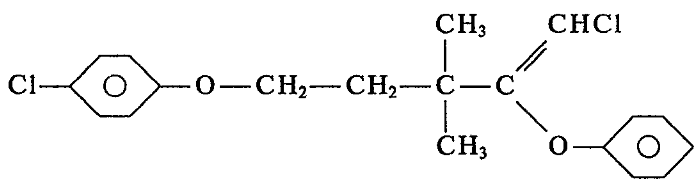

116 g (1 Mol) Natriumphenolat und 147 g (0,5 Mol) 5-(4-Chlorphenoxy)-1,1-dichlor-3,3-dimethyl-1-penten werden in 500 ml Dimethylformamid 10 Stunden unter Rückfluß erhitzt. Man verdünnt mit Methylenchlorid und schüttelt mit 2 n Natronlauge aus. Die getrocknete organische Phase wird am Rotationsverdampfer bei 0,1 mbar und 150°C vom Lösungsmittel befreit. Es bleiben 158 g (0,45 Mol; 90% der Theorie) 1-Chlor-5-(4-chlorphenoxy)-3,3-dimethyl-2-phenoxy-1-penten als Öl zurück.

NMR ($CDCl_3$): $\delta =$ 1,2 (s, 6 H), 2,0 (t, 2 H, J = 7 Hz), 4,0 (t, 2 H, J = 7 Hz), 5,9 (s, 1 H), 6,7—7,4 ppm (m, 9 H).

3. Stufe

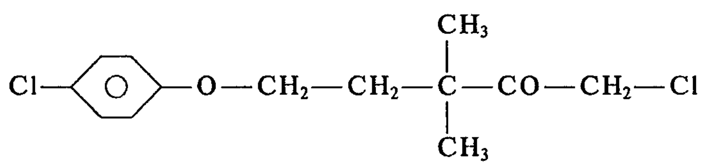

351 g (1 Mol) 1-Chlor-5-(4-chlorphenoxy)-3,3-dimethyl-2-phenoxy-1-penten werden in 600 ml Ethanol und 200 ml konzentrierter Salzsäure 4 Stunden unter Rückfluß erhitzt. Man zieht den Alkohol

im Vakuum ab, verdünnt mit Methylenchlorid und schüttelt einmal mit Wasser und dreimal mit verdünnter Notronlauge aus. Die getrocknete Lösung wird im Vakuum von restlichem Lösungsmittel befreit. Es bleiben 239 g (0,89 Mol, 89 % der Theorie) 1-Chlor-5-(4-chlorphenoxy)-3,3-dimethyl-pentan-2-on als Öl zurück.

NMR ($CDCl_3$): $\delta =$ 1,25 (s, 6 H), 2,1 (t, 2 H, J = 6 Hz), 3,9 (t, 2 H, J = 6 Hz), 4,45 (s, 2 H), 6,7–7,3 ppm (m, 4 H).

## Patentansprüche

1. Verfahren zur Herstellung von 5-Aryloxy-1-chlor-3,3-dimethyl-pentan-2-onen der Formel

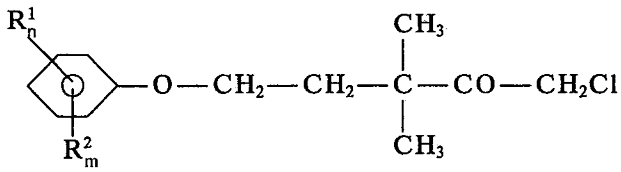

(I)

in welcher

$R^1$ und $R^2$ gleich oder verschieden sind und für Halogen, Alkyl mit 1 bis 4 Kohlenstoffatomen, Alkoxy und Alkylthio mit jeweils 1 bis 2 Kohlenstoffatomen, Halogenalkyl, Halogenalkoxy und Halogenalkylthio mit jeweils 1 bis 2 Kohlenstoffatomen und 1 bis 5 gleichen oder verschiedenen Halogenatomen stehen, für Cyclohexyl, für Dialkylamino mit 1 bis 4 Kohlenstoffatomen in jedem Alkylteil und weiterhin für Nitro, Cyano, sowie Alkoxycarbonyl mit 1 bis 4 Kohlenstoffatomen im Alkylteil und für gegebenenfalls durch Halogen substituiertes Phenyl stehen, und

n und m gleich oder verschieden sind und für die Werte 0, 1, 2, 3, 4 oder 5 stehen, mit der Einschränkung, daß die Summe von n und m nicht größer als 5 ist,

dadurch gekennzeichnet, daß man 1,1,5-Trichlor-3,3-dimethyl-1-penten der Formel

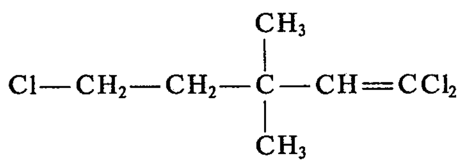

(II)

mit Phenolen der Formel

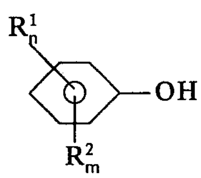

(III)

in welcher
$R^1$, $R^2$, n und m die obigen Bedeutungen haben,
in Gegenwart einer Base in einem Verdünnungsmittel zu Verbindungen der Formel

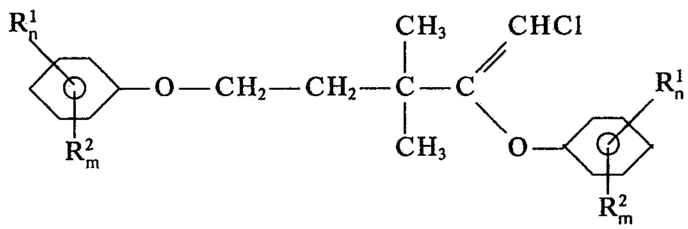

(IV)

in welcher
$R^1$, $R^2$, n und m die obige Bedeutung haben,
umsetzt und dieses Reaktionsprodukt einer sauren Hydrolyse unterwirft.

2. Verfahren nach Anspruch 1, dadurch gekennzeichnet, daß man eine Base aus der Reihe Alkali- oder Erdalkali-hydroxide oder -carbonate oder Alkali-alkoholate verwendet.

3. Verfahren nach Anspruch 1, dadurch gekennzeichnet, daß man die Umsetzung der Verbindung der

Formel (II) mit dem Phenol der Formel (III) im Temperaturbereich zwischen +100 und 250°C und die anschließende Hydrolyse im Temperaturbereich zwischen +20 und 150°C durchführt.

4. Verfahren nach Ansprüchen 1 und 3, dadurch gekennzeichnet, daß man die erste Umsetzungsstufe [(II) mit (III)] im Temperaturbereich zwischen +120 und 220°C und die zweite Stufe (Hydrolyse) im Temperaturbereich zwischen +40 und 100°C durchführt.

5. Verfahren nach Anspruch 1, dadurch gekennzeichnet, daß man die Hydrolyse mit Mineralsäuren ausführt.

6. Verfahren nach Ansprüchen 1 und 5, dadurch gekennzeichnet, daß man die Hydrolyse mit einer Mineralsäure und einer derselben beigefügten organischen Säure ausführt.

7. Verfahren nach Anspruch 1, dadurch gekennzeichnet, daß man die Umsetzung von 1,1,5-Trichlor-3,3-dimethyl-1-penten der Formel (II) mit einem Phenol der Formel (III) und die Hydrolyse von den Verbindungen der Formel (IV) in einem Eintopfverfahren zusammenfaßt.

8. Verfahren nach Anspruch 1, dadurch gekennzeichnet, daß man zunächst das 1,1,5-Trichlor-3,3-dimethyl-1-penten der Formel (II) mit einem Äquivalent eines Phenols der Formel (III) in Gegenwart einer Base zu einer Verbindung der Formel

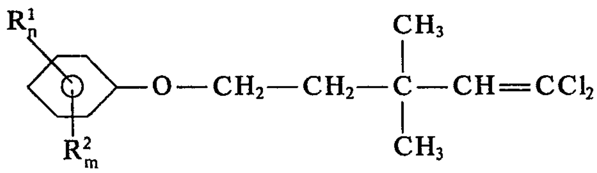
(V)

in welcher
$R^1$, $R^2$, n und m die in Anspruch 1 genannte Bedeutung haben,
umsetzt und in einer zusätzlichen Stufe mit einem Phenolat der Formel

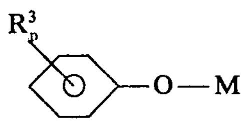
(VI)

in welcher

$R^3$ für Halogen und für Alkyl mit 1 bis 2 Kohlenstoffatomen steht,
p für die Zahlen 0, 1, 2 oder 3 steht, und
M für ein Äquivalent eines Alkali- oder Erdalkalimetallions steht,

zur Reaktion bringt, wobei man die anschließend erhaltenen Verbindungen der Formel

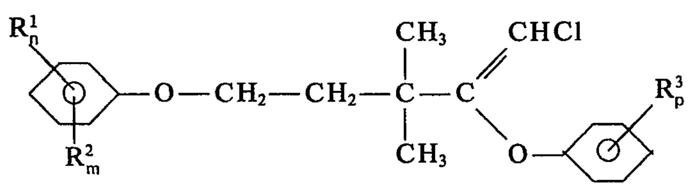
(VII)

in welcher
$R^1$, $R^2$, $R^3$, n, m und p die oben erwähnten Bedeutungen haben,
einer sauren Hydrolyse unterwirft.

**Claims**

1. Process for the preparation of 5-aryloxy-1-chloro-3,3-dimethyl-2-pentanones of the formula

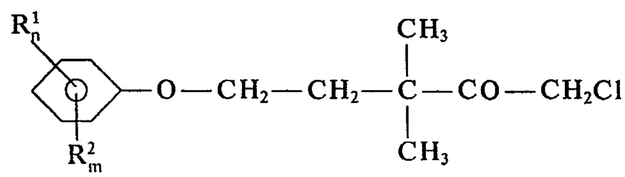
(I)

in which

$R^1$ and $R^2$ are identical or different and represent halogen, alkyl having 1 to 4 carbon atoms, alkoxy and alkylthio, each having 1 to 2 carbon atoms, halogenoalkyl, halogenoalkoxy and halo-

genoalkylthio, each having 1 to 2 carbon atoms and 1 to 5 identical or different halogen atoms, or represent cyclohexyl, dialkylamino having 1 to 4 carbon atoms in each alkyl moiety, and also nitro, cyano and alkoxycarbonyl having 1 to 4 carbon atoms in the alkyl moiety and phenyl which is optionally substituted by halogen, and

n and m are identical or different and represent the numbers 0, 1, 2, 3, 4 or 5, with the proviso that the sum of n and m is not greater than 5,

characterised in that 1,1,5-trichloro-3,3-dimethyl-1-pentene of the formula

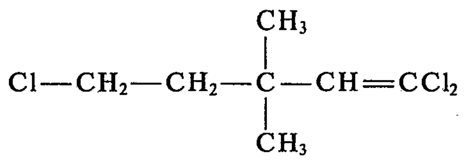

(II)

is reacted with phenols of the formula

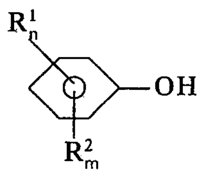

(III)

in which
$R^1$, $R^2$, n and m have the above meanings,
in the presence of a base in a diluent, to give compounds of the formula

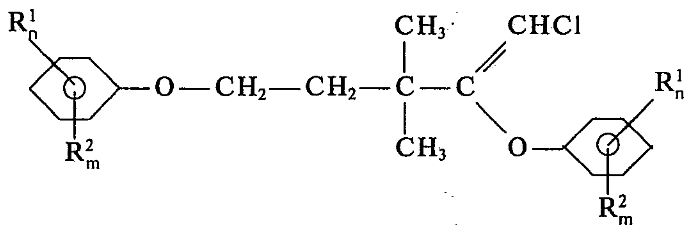

(IV)

in which
$R^1$, $R^2$, n and m have the above meaning,
and this reaction product is subjected to acid hydrolysis.

2. Process according to Claim 1, characterised in that a base from the series comprising alkali metal or alkaline earth metal hydroxides or carbonates or alkali metal alcoholates is used.

3. Process according to Claim 1, characterised in that the reaction of the compound of the formula (II) with the phenol of the formula (III) is carried out in the temperature range between +100 and 250°C and the subsequent hydrolysis is carried out in the temperature range between +20 and 150°C.

4. Process according to Claims 1 and 3, characterised in that the first reaction step [(II) with (III)] is carried out in the temperature range between +120 and 220°C, and the second step (hydrolysis) is carried out in the temperature range between +40 and 100°C.

5. Process according to Claim 1, characterised in that the hydrolysis is carried out with mineral acids.

6. Process according to Claims 1 and 5, characterised in that the hydrolysis is carried out with a mineral acid and an organic acid added to the latter.

7. Process according to Claim 1, characterised in that the reaction of 1,1,5-trichloro-3,3-dimethyl-1-pentene of the formula (II) with a phenol of the formula (III) and the hydrolysis of the compounds of the formula (IV) are united in a one-pot process.

8. Process according to Claim 1, characterised in that initially the 1,1,5-trichloro-3,3-dimethyl-1-pentene of the formula (II) is reacted with one equivalent of a phenol of the formula (III) in the presence of a base, to give a compound of the formula

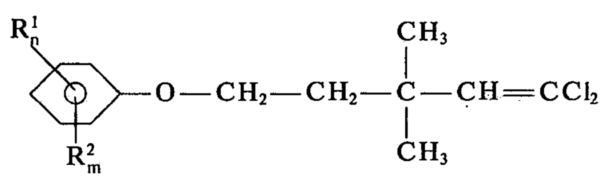

(V)

in which
$R^1$, $R^2$, n and m have the meaning mentioned in Claim 1,

and, in an additional step, is brought to reaction with a phenolate of the formula

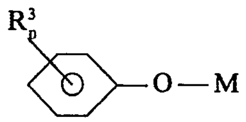

(VI)

in which

$R^3$ represents halogen and alkyl having 1 to 2 carbon atoms,
p represents the figures 0, 1, 2 or 3, and
M represents one equivalent of an alkali metal or alkaline earth metal ion,

the subsequently obtained compounds of the formula

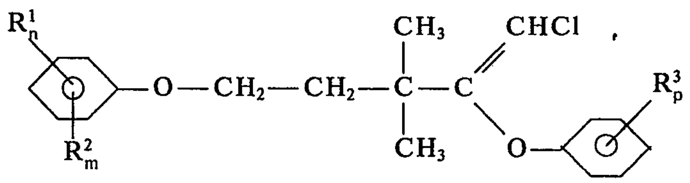

(VII)

in which
$R^1$, $R^2$, $R^3$, n, m and p have the meanings mentioned above,
being subjected to acid hydrolysis.

**Revendications**

1. Procédé de fabrication de 5-aryloxy-1-chloro-3,3-diméthylpentane-2-ones de formule:

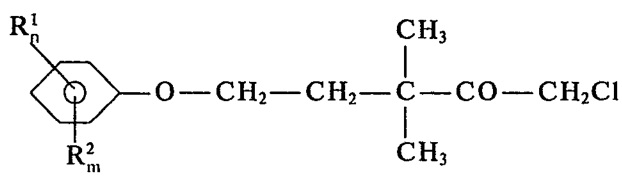

(I)

dans laquelle

$R^1$ et $R^2$ sont identiques ou différents et représentent de l'halogène, un alcoyle ayant 1 à 4 atomes de carbone, alcoxy et alcoylthio ayant chacun 1 à 2 atomes de carbone, halogénoalcoyle, halogénoalcoxy et halogénoalcoylthio ayant chacun 1 à 2 atomes de carbone et 1 à 5 atomes d'halogène identiques ou différents, cyclohexyle, dialcoylamino ayant 1 à 4 atomes de carbone dans chaque portion alcoyle et, en outre, nitro, cyano de même que alcoxycarbonyle ayant 1 à 4 atomes de carbone dans la portion alcoyle et phényle éventuellement substitué par de l'halogène, et

n et m sontidentiques ou différents et présentent les valeurs 0, 1, 2, 3, 4 ou 5, avec la limitation que la somme de n et de m ne doit pas être supérieure à 5,

caractérisé en ce qu'on fait réagir du 1,1,5-trichloro-3,3-diméthyl-1-pentène de formule:

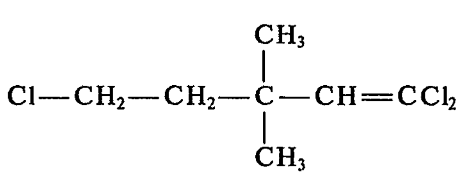

(II)

avec des phénols de formule:

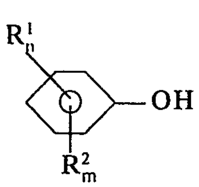

(III)

dans laquelle
$R^1$, $R^2$, n et m ont la signification indiquée plus haut,
en présence d'une base dans un diluant pour donner des composés de formule:

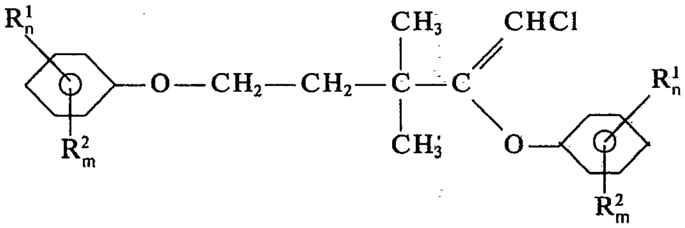

(IV)

dans laquelle
$R^1$, $R^2$, n et m ont la signification indiquée plus haut,
et en ce qu'on soumet ce produit de réaction à une hydrolyse acide.

2. Procédé selon la revendication 1, caractérisé en ce qu'on utilise une base de la série des hydroxydes ou carbonates alcalins ou alcalino-terreux ou des alcoolates alcalins.

3. Procédé selon la revendication 1, caractérisé en ce qu'on exécute la réaction du composé de formule (II) avec le phénol de formule (III) dans l'intervalle de température compris entre +100 et 250°C et l'hydrolyse consécutive dans l'intervalle de température compris entre +20 et 150°C.

4. Procédé selon les revendications 1 et 3, caractérisé en ce qu'on exécute le premier stade de réaction de (II) avec (III) dans l'intervalle de température compris entre +120 et 220°C et le second stade (hydrolyse) dans l'intervalle de température compris entre +40 et 100°C.

5. Procédé selon la revendication 1, caractérisé en ce qu'on exécute l'hydrolyse avec des acides minéraux.

6. Procédé selon les revendications 1 à 5, caractérisé en ce qu'on exécute l'hydrolyse avec un acide minéral en plus d'un acide organique ajouté à celui-ci.

7. Procédé selon la revendication 1, caractérisé en ce qu'on réunit la réaction du 1,1,5-trichloro-3,3-diméthyl-1-pentène de formule (II) avec un phénol de formule (III) et l'hydrolyse des composés de formule (IV) en un procédé dans un seul récipient.

8. Procédé selon la revendication 1, caractérisé en ce qu'on fait réagir tout d'abord le 1,1,5-trichloro-3,3-diméthyl-1-pentène de formule (II) avec un équivalent d'un phénol de formule (III) en présence d'une base pour donner un composé de formule

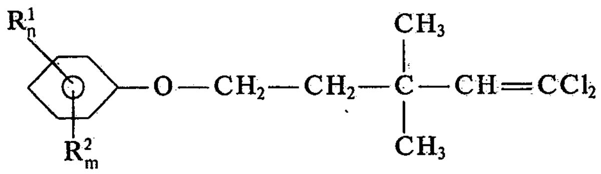

(V)

dans laquelle
$R^1$, $R^2$, n et m ont la signification citée à la revendication 1,
et en ce que, dans un stade supplémentaire, on fait réagir ce dernier avec un phénolate de formule:

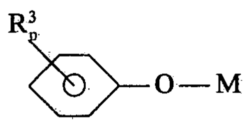

(VI)

dans laquelle

$R^3$ représente de l'halogène et un alcoyle ayant 1 à 2 atomes de carbone,
p représente les nombres 0, 1, 2 ou 3 et
M représente un équivalent d'un ion de métal alcalin ou alcalino-terreux,

et l'on soumet les composés consécutivement obtenus, de formule:

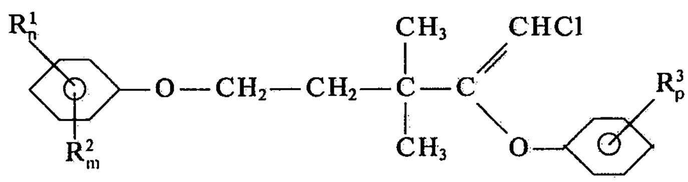

(VII)

dans laquelle $R^1$, $R^2$, $R^3$, n, m et p ont la signification indiquée plus haut, à une hydrolyse acide.